(19) Europäisches Patentamt
European Patent Office
Office européen des brevets

(11) EP 0 714 654 B1

(12) **EUROPÄISCHE PATENTSCHRIFT**

(45) Veröffentlichungstag und Bekanntmachung des Hinweises auf die Patenterteilung:
**21.03.2001 Patentblatt 2001/12**

(51) Int Cl.$^7$: **A61K 7/06**

(21) Anmeldenummer: **95111941.1**

(22) Anmeldetag: **29.07.1995**

(54) **Mittel und Verfahren zur dauerhaften Haarverformung**

Composition and process for permanent waving of hair

Composition et procédé pour la déformation permanente des cheveux

(84) Benannte Vertragsstaaten:
**DE ES FR GB IT**

(30) Priorität: **03.12.1994 DE 4443062**

(43) Veröffentlichungstag der Anmeldung:
**05.06.1996 Patentblatt 1996/23**

(73) Patentinhaber: **Wella Aktiengesellschaft**
**64274 Darmstadt (DE)**

(72) Erfinder:
- **Lang, Günther, Dr.**
  **D-64354 Reinheim (DE)**
- **Uhl, Kirstin**
  **D-64297 Darmstadt (DE)**
- **Sendelbach, Gerhard, Dr.**
  **D-64297 Darmstadt (DE)**
- **Maresch, Gerhard**
  **D-64295 Darmstadt (DE)**

(56) Entgegenhaltungen:
**EP-A- 0 530 974** **WO-A-94/08556**
**WO-A-94/21224** **DE-C- 3 719 086**
**GB-A- 2 197 352**

- **SEIFEN ÖLE FETTE WACHSE, Bd. 115, Nr. 17, 31.Oktober 1989 AUGSBURG (DE), Seiten 607-612, XP 000084376 SACKLOWSKI, H. 'Organomodifizierte Polydimethylsiloxane in der Haarpflege'**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Die vorliegende Erfindung betrifft diquaternäre Polysiloxane enthaltende Dauerverformungsmittel sowie ein Verfahren zur dauerhaften Haarverformung unter Verwendung dieser Mittel.

Haare sind in ihrer morphologischen Struktur vom Haaransatz bis zu den Haarspitzen unterschiedlich. Dies beruht unter anderem auf Umwelteinflüssen (beispielsweise der Sonneneinwirkung oder anderen Witterungseinflüssen) sowie chemischen, mechanischen Behandlungen (beispielsweise Kämmen oder Fönen, Dauerwell- oder Färbebehandlungen, Haarwäsche), denen das Haar im Laufe seiner Lebenszeit unterworfen ist. Diese äußeren Einwirkungen sowie alterungsbedingte morphologische Veränderungen der Haare bewirken eine erhöhte statische Aufladung der Haare, eine verschlechterte Kämmbarkeit, einen rauhen und unangenehmen Griff, einen verminderten Glanz sowie eine zunehmende Sprödigkeit der Haare und können im Extremfall zu einem Bruch der Haare während oder nach einer Dauerverformungsbehandlung führen.

Es wurden bereits mehrere Versuche unternommen diese Probleme zu lösen. So wurde zum Beispiel die Verwendung von im sauren bis neutralen pH-Bereich einsetzbaren Dauerverformungsmitteln auf Thioglykolsäureesterbasis empfohlen. Durch die Verwendung von Dauerverformungsmitteln auf Thioglykolsäureesterbasis wird zwar eine relativ gleichmäßige und schonende Verformung der Haare vom Haaransatz bis zu den Haarspitzen erzielt, jedoch sind die physiologischen und insbesondere sensibilisierenden Eigenschaften der Thioglykolsäureester nicht befriedigend, so daß der Einsatz von anderen physiologisch besser verträglichen Reduktionsmitteln anstelle der Thioglykolsäureester wünschenswert ist. Ebenfalls wird in der Literatur empfohlen, Dauerverformungsmitteln kationische Polymere und/oder kationische Tenside zuzusetzen, wodurch eine gute Kämmbarkeit der Haare und ein angenehmer Griff der Haare erzielt werden soll. Es hat sich jedoch gezeigt, daß bei Verwendung dieser Mittel eine ungleichmäßige Verformung der Haare (starke Verformung im Bereich der Haarspitzen/schwache Verformung im Bereich des Haaransatzes) erhalten wird.

So betrifft z. B. die GB-A 2 197 352 ein Dauerwellmittel, welches (A) eine Aminosäure und (B) eine wasserlösliche oder emulgierbare Silikonverbindung in einer Menge enthält, welche ausreicht, um eine verbesserte Wellstabilität zu liefern. Eine unter dem Handelsnamen ABIL ®B 9905 von der Herstellerin Th. Goldschmidt AG vertriebene geeignete monoquatemäre Silikonverbindung ist auf der Seite 4 Zeilen 37 bis 55 als Strukturformel angeführt. Für die Aufgabenstellung, die Wellstabilität zu verbessern, werden bevorzugt nichtionische und wasserlösliche Dimethicon Copolyole empfohlen (vgl. S.3 Z.32 bis S.4 Z.35).

Des weiteren wird in der WO-A 94/21224 die Verwendung von nichtflüchtigen Organopolysiloxanen in Kombination mit Trimethylammoniummethacrylathomopolymer oder dessen Copolymer mit Acrylamid in kosmetischen Mitteln zur Haut-und Haarbehandlung empfohlen (Anspruch 1). Die Haarbehandlungsmittel können als Shampoos oder Spülungen u. a. vor, nach oder zwischen einer Dauerwelle angewandt werden (vgl. S.7 Z. 26- 33). Als geeignete nichtflüchtige Organopolysiloxane wird eine große Anzahl sehr unterschiedlicher nichtionischer und kationischer Verbindungen genannt (vgl. S.2 Z.33 bis S.6 Z.12). Als Beispiele für Organopolysiloxane mit quaternären Gruppen werden u. a. auf der Seite 6 im Absatz 3 unter h) die Handelsbezeichnungen X28108, X28109 und ABIL K 3270 angeführt.

Die DE-C 37 19 086 betrifft die quaternären Organopolysiloxane mit zwei endständigen Ammoniumgruppen der Formel (I) der vorliegenden Anmeldung, ihre Herstellung sowie deren Verwendung in kosmetischen Zubereitungen zur Pflege der Haare (vgl. Ansprüche 1 bis 3). Die dort gestellte Aufgabe liegt darin, Verbindungen aufzufinden mit verbesserten Gebrauchseigenschaften, welche insbesondere die Pflege der Haare verbessern (vgl. S.5 Z.22-23). Als weiterer Vorteil finden sich noch auf der Seite 12 in Zeile 24, die verbesserte Naß- und Trockenkämmbarkeit sowie verbesserter Glanz der Haare. Die Verwendung bei der Dauerwelle wird nicht erwähnt; dementsprechend wird auch das Problem der ungleichmäßigen Dauerverformung vom Haaransatz zu den Haarspitzen nicht erörtert.

Es bestand daher die Aufgabe, ein Dauerverformungsverfahren sowie geeignete Mittel zur Durchführung dieses Verfahrens zur Verfügung zu stellen, durch die eine schonende und gleichmäßige Dauerverformung der Haare vom Haaransatz bis zu den Haarspitzen bei gleichzeitig guter physiologischer Verträglichkeit (insbesondere bezüglich des Sensibilisierungsrisikos) ermöglicht wird.

Überraschenderweise wurde nunmehr gefunden, daß die vorstehend beschriebene Aufgabe in hervorragender Weise durch die Verwendung von diquaternäre Polysiloxane enthaltenden Dauerverformungsmitteln gelöst wird.

Gegenstand der vorliegenden Erfindung ist daher ein Mittel zur dauerhaften Verformung von Haaren auf der Basis einer keratinreduzierenden Verbindung, welches
dadurch gekennzeichnet ist, daß es ein diquaternäres Polysiloxan der allgemeinen Formel (I)

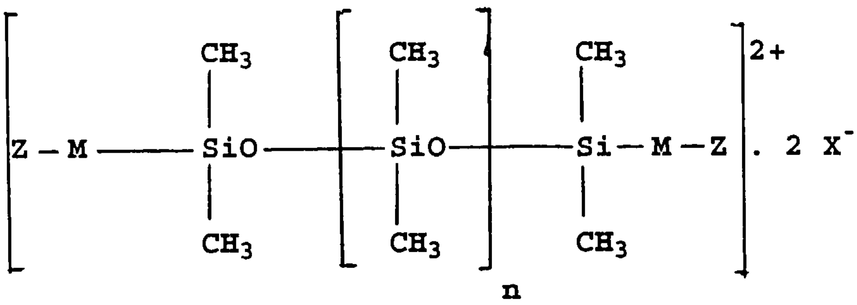 (I),

wobei
Z der Rest

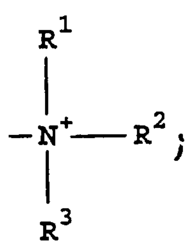 ; 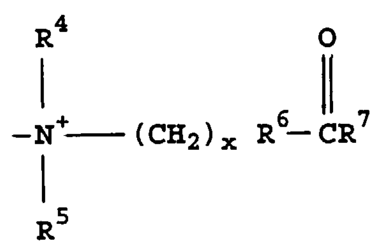 oder 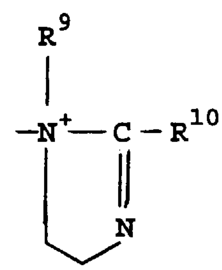

ist,

$R^1$, $R^2$, $R^3$ = Alkylreste mit 1 bis 22 Kohlenstoffatomen oder Alkenylreste mit 2 bis 22 Kohlenstoffatomen, wobei die Alkyl- oder Alkenylreste Hydroxylgruppen aufweisen können und mindestens einer der Reste $R^1$, $R^2$, $R^3$ mindestens 10 Kohlenstoffatome aufweist,

$R^4$, $R^5$, $R^7$, $R^9$, $R^{10}$ = Alkylreste mit 1 bis 22 Kohlenstoffatomen oder Alkenylreste mit 2 bis 22 Kohlenstoffatomen, wobei die Alkyl- oder Alkenylreste Hydroxylgruppen aufweisen können,

$R^6$ = -O- oder $NR^8$-Rest, $R^8$ = Alkyl- oder Hydroxyalkylrest mit 1 bis 4 Kohlenstoffatomen oder Wasserstoffrest,

x = 2 bis 4,

M = ein zweiwertiger Rest, ausgewählt aus der Gruppe

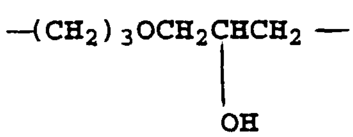 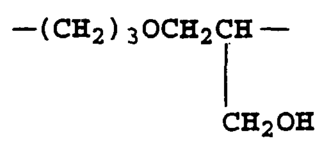

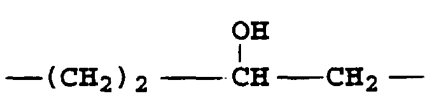 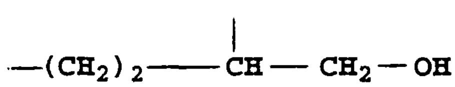

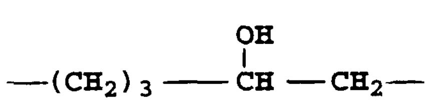 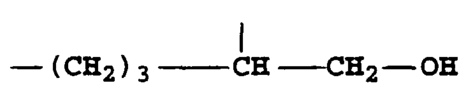

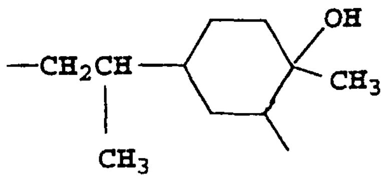

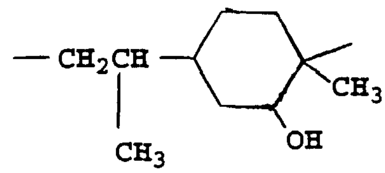

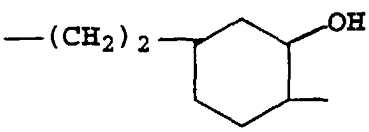

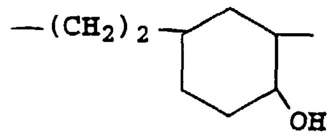

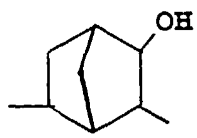

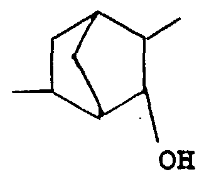

wobei das N-Atom des Restes Z mit dem Rest M über das zur C-OH-Gruppe im Rest M benachbarte Kohlenstoffatom verbunden ist,

n = eine Zahl von 0 bis 200,
$X^-$ = anorganisches oder organisches Anion, das von einer üblichen physiologisch verträglichen Säure HX herrührt,

enthält.

Unter den diquaternären Polysiloxanen der Formel (I) sind die Verbindungen besonders bevorzugt, bei denen gilt:

M = $-(CH_2)_3-O-CH_2-CH(OH)-CH_2-$;

Z = 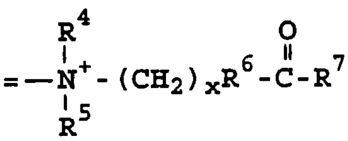 ;

$R^4$, $R^5$ = $CH_3$;
$R^6$ = NH-Rest; $R^7$ = Kokosfettsäurerest; x = 3; n = 10 oder 30 und $X^-$ = Acetatanion.

Die Zusammensetzung des erfindungsgemäßen Dauerverformungsmittels entspricht den für derartige Mittel jeweils bekannten Zubereitungsarten auf der Basis von keratinreduzierenden Verbindungen.

Das diquaternäre Polysiloxan der Formel (I) ist in diesem Dauerverformungsmittel vorzugsweise in einer Menge von 0,02 bis 5 Gewichtsprozent (bezogen auf die Gesamtmenge der gebrauchsfertigen Zubereitung) enthalten, wobei eine Menge von 0,05 bis 5 Gewichtsprozent besonders bevorzugt ist.

Das erfindungsgemäße Dauerverformungsmittel kann in einer Vielzahl von Zubereitungsarten verwendet werden, von denen nachstehend einige bevorzugte Zubereitungsarten näher erläutert werden sollen.

Bei dem Mittel zur dauerhaften Verformung von Haaren handelt es sich insbesondere um wäßrige,schwach sauer bis alkalisch (pH = 6 bis 10) eingestellte Zubereitungen, welche eine keratinreduzierende Verbindung, beispielsweise Mercaptocarbonsäuren wie Thioglykolsäure und Thiomilchsäure oder deren Salze und 2-Hydroxy-3-mercaptopropionsäure, Cystein oder dessen Salze und Derivate; Cysteamin oder dessen Salze und Derivate sowie Schweflige Säure oder deren Salze, alleine oder in Kombination miteinander, enthalten. Die Einsatzkonzentration der keratinreduzierenden Verbindung beträgt hierbei etwa 2 bis 25 Gewichtsprozent, vorzugsweise 5 bis 18 Gewichtsprozent. Der erforderliche pH-Wert wird überwiegend durch die Zugabe von Ammoniak und organischen Aminen sowie Ammonium- und Alkalicarbonat oder -hydrogencarbonat eingestellt.

Das Mittel zur dauerhaften Verformung von Haaren kann sowohl in Form einer wäßrigen Lösung oder Emulsion als auch in verdickter Form auf wäßriger Basis, insbesondere als Gel, Creme oder Paste, vorliegen.

Selbstverständlich kann das Verformungsmittel alle für derartige Mittel üblichen und bekannten Zusatzstoffe, zum Beispiel Verdickungsmittel, wie beispielsweise Kaolin, Bentonit, Fettsäuren, höhere Fettalkohole, Stärke, Polyacrylsäure und deren Derivate, Cellulosederivate, Alginate, Vaseline oder Paraffinöl; Netzmittel oder Emulgatoren aus den Klassen der anionischen, kationischen, amphoteren oder nichtionogenen oberflächenaktiven Substanzen, beispielsweise Fettalkoholsulfate, Fettalkoholethersulfate, Alkylsulfonate, Alkylbenzolsulfate, quaternäre Ammoniumsalze, Alkylbetaine, oxethylierte Fettsäurester; ferner Trübungsmittel, wie zum Beispiel Polyethylenglykolester; oder Alkohole, wie beispielsweise Ethanol, Propanol, Isopropanol oder Glycerin, Lösungsvermittler; Stabilisatoren; Puffersubstanzen; Parfümöle, Farbstoffe sowie haarkonditionierende und haarpflegende Bestandteile, wie zum Beispiel Lanolinderivate, Cholesterin, Pantothensäure oder Betain, enthalten. Die erwähnten Bestandteile werden in den für solche Zwecke üblichen Mengen verwendet, zum Beispiel die Netzmittel und Emulgatoren in Konzentrationen von 0,2 bis 30 Gewichtsprozent, während die Verdickungsmittel in einer Menge von 0,5 bis 20 Gewichtsprozent in diesem Mittel enthalten sein können.

Weiterhin können diesem Mittel zur Wirkungssteigerung sogenannte Quell- und Penetrationsstoffe, wie zum Beispiel Dipropylenglykolmonomethylether, 2-Pyrrolidon oder Imidazolidin-2-on, in einer Menge von 0,5 bis 20 Gewichtsprozent sowie zur Vermeidung einer Überkrausung der Haare Dithioverbindungen, beispielsweise Dithiodiglykolsäure, Dithiodimilchsäure oder deren Salze, zugesetzt werden.

Durch Variation des pH-Wertes kann ein Mittel zur Verfügung gestellt werden, das universell für jede Haarstruktur, gegebenenfalls unter zusätzlicher Wärmeeinwirkung, geeignet ist. Das Mittel bewirkt eine elastische, dauerhafte und gleichmäßige Verformung der Haare vom Haaransatz bis zur Haarspitze ohne allergische oder sensibilisierende Reaktionen hervorzurufen. Insbesondere wird die Verformung im Bereich des Haaransatzes verbessert und eine Überkrausung der Haarspitzen im Vergleich zum Haaransatz vermieden sowie der Glanz und die Kämmbarkeit der Haare verbessert.

Die vorliegende Erfindung betrifft weiterhin ein Verfahren zur dauerhaften Verformung von Haaren, bei dem man das Haar bevor und/oder nachdem man es in die gewünschte Form bringt, mit einem Verformungsmittel behandelt, mit Wasser spült, sodann oxidativ nachbehandelt, mit Wasser spült, gegebenenfalls zur Wasserwelle legt und sodann trocknet, welches dadurch gekennzeichnet ist, daß als Verformungsmittel das vorstehend beschriebene erfindungsgemäße Mittel verwendet wird.

Gemäß dem erfindungsgemäßen Verfahren kann das Haar zunächst mit einem Shampoo gewaschen und danach mit Wasser gespült werden. Anschließend wird das handtuchtrockene Haar in einzelne Strähne aufgeteilt und auf Wickler mit einem Durchmesser von 5 bis 30 Millimetern, bevorzugt 5 bis 15 Millimeter, gewickelt. Sodann wird das Haar mit einer für die Haarverformung ausreichenden Menge, vorzugsweise 60 bis 120 Gramm, des beschriebenen erfindungsgemäßen Verformungsmittels behandelt.

Nach einer für die dauerhafte Verformung des Haares ausreichende Einwirkungszeit, welche je nach Haarbeschaffenheit, dem pH-Wert und der Verformungswirksamkeit des Verformungsmittels sowie in Abhängigkeit von der Anwendungstemperatur 5 bis 30 Minuten (10 bis 30 Minuten ohne Wärmeeinwirkung; 5 bis 20 Minuten mit Wärmeeinwirkung) beträgt, wird das Haar mit Wasser gespült und sodann oxidativ nachbehandelt ("fixiert"). Das Nachbehandlungsmittel wird, je nach Haarfülle, vorzugsweise in einer Menge von 80 bis 100 Gramm, verwendet.

Für die oxidative Nachbehandlung kann jedes beliebige, bisher für eine derartige Behandlung verwendete, Nachbehandlungsmittel verwendet werden. Beispiele für in solchen Nachbehandlungsmitteln verwendbare Oxidationsmittel sind Kalium- und Natriumbromat, Natriumperborat, Harnstoffperoxid und Wasserstoffperoxid. Die Konzentration des Oxidationsmittels ist in Abhängigkeit von der Anwendungszeit (in der Regel etwa 5 bis 15 Minuten) und der Anwendungstemperatur unterschiedlich. Normalerweise liegt das Oxidationsmittel in dem gebrauchsfertigen wäßrigen Nachbehandlungsmittel in einer Konzentration von 0,5 bis 10 Gewichtsprozent vor. Das Mittel für die oxidative Nachbehandlung kann selbstverständlich weitere Stoffe, wie zum Beispiel Netzmittel, Pflegestoffe wie kationaktive Polymere, schwache Säuren, Puffersubstanzen oder Peroxidstabilisatoren, enthalten und in Form einer wäßrigen Lösung, einer Emulsion sowie in verdickter Form auf wäßriger Basis, insbesondere als Creme, Gel oder Paste, vorliegen.

Anschließend werden die Wickler entfernt. Falls erforderlich, kann das abgewickelte Haar nun nochmals oxidativ nachbehandelt werden. Sodann wird das Haar mit Wasser gespült, gegebenenfalls zur Wasserwelle gelegt und schließlich getrocknet.

Die nachfolgenden Beispiele sollen die Erfindung näher erläutern, ohne diese auf die Beispiele beschränken zu wollen.

| Beispiel 1: Dauerverformungsmittel für normales Haar | |
|---|---|
| 11,9 g | Ammoniumthioglykolat |
| 5,0 g | Ammoniumhydrogencarbonat |
| 1,0 g | mit 4 Mol Ethylenoxid oxethylierter Lauryl-alkohol |
| 0,5 g | Ammoniak (25-%ige wäßrige Lösung) |
| 0,5 g | Cocoamidopropylbetain |
| 0,5 g | Parfümöl |
| 2,0 g | diquaternäres Polysiloxan gemäß Formel (I) (Handelsname:: ABIL® -QUAT 3270 der Firma Th. Goldschmidt AG, Essen/BRD) |
| 0,1 g | Vinylpyrrolidon/Styrol-Mischpolymerisat (Handelsname:: ANTARA® 430 der GAF Corp., New York/ USA) |
| 78,5 g | Wasser |
| 100,0 g | |

Der pH-Wert dieses Dauerverformungsmittels beträgt 8,3. Normales, nicht vorgeschädigtes Haar wird gewaschen, mit einem Handtuch frottiert und sodann auf Wickler mit einem Durchmesser von 6 Millimetern gewickelt. Anschließend wird das Haar mit dem vorstehend beschriebenen Dauerverformungsmittel gleichmäßig durchfeuchtet und nach einer Einwirkungszeit von 18 Minuten bei Raumtemperatur gründlich mit Wasser ausgespült. Sodann wird das Haar mit 80 Gramm einer 3-prozentigen wäßrigen Wasserstoffperoxidlösung oxidativ nachbehandelt, erneut mit Wasser ausgespült, zur Wasserwelle gelegt und getrocknet. Das so behandelte Haar besitzt eine sehr gleichmäßige und lebhafte Krause.

| Beispiel 2: Dauerverformungsmittel für normales und schwer verformbares Haar | |
|---|---|
| 14,2 g | Ammoniumthioglykolat |
| 5,5 g | Ammoniumhydrogencarbonat |
| 3,0 g | 1,2-Propandiol |
| 1,2 g | mit 40 Mol Ethylenoxid oxethyliertes Rizinusöl |
| 1,0 g | Ammoniak (25-%ige wäßrige Lösung) |
| 0,8 g | Parfümöl |
| 0,5 g | Poly (dimethyldiallylammoniumchlorid) |
| 0,3 g | Vinylpyrrolidon/Styrol-Mischpolymerisat (Handelsname: ANTARA® 430 der GAF Corp., New York/ USA) |
| 1,0 g | diquaternäres Polysiloxan der Formel (I) (Handelsname: ABIL®-QUAT 3270 der Firma Th. Goldschmidt AG, Essen/BRD) |
| 72,5 g | Wasser |
| 100,0 g | |

Der pH-Wert dieses Mittels beträgt 8,5.

Die Anwendung des Dauerverformungsmittels erfolgt in der in Beispiel 2 beschriebenen Weise, wobei die Einwirkungszeit des Dauerverformungsmittels jedoch 20 Minuten beträgt. Es wird eine natürlich wirkende, gleichmäßige Verformung der Haare vom Haaransatz bis zu den Haarspitzen erhalten.

| Beispiel 3: Dauerverformungsmittel für gefärbtes Haar | |
|---|---|
| 9,1 g | Ammoniumthioglykolat |
| 2,5 g | Ammoniumhydrogencarbonat |
| 1,0 g | mit 40 Mol Ethylenoxid oxethyliertes Rizinusöl |
| 1,0 g | Poly(dimethyldiallylammoniumchlorid) |
| 1,0 g | Cocobetaine |
| 0,6 g | Parfümöl |
| 0,4 g | Ammoniak (25-%ige wäßrige Lösung) |
| 3,0 g | diquaternäres Polysiloxan der Formel (I) (Handelsname:: ABIL® -QUAT 3270 der Firma Th. Goldschmidt AG, Essen/BRD) |

(fortgesetzt)

| Beispiel 3: Dauerverformungsmittel für gefärbtes Haar | |
|---|---|
| 81,4 g | Wasser |
| 100,0 g | |

Der pH-Wert dieses Mittels beträgt 8,0.

Durch Färbebehandlungen vorgeschädigtes Haar wird mit einem milden Shampoo gewaschen, mit einem Handtuch getrocknet und auf Wickler mit einem Durchmesser von 8 Millimetern gewickelt. Anschließend wird das vorstehend beschriebene Dauerverformungsmittel gleichmäßig auf dem gewickelten Haar verteilt. Nach einer Einwirkungszeit von 15 Minuten bei Raumtemperatur wird das gewickelte Haar mit Wasser ausgespült und mit einem Handtuch leicht angetrocknet. Sodann wird das gewickelte Haar mit einer 2,5-prozentigen wäßrigen Wasserstoffperoxidlösung oxidativ nachbehandelt. Nach einer Einwirkungszeit von 5 Minuten werden die Wickler entfernt, die Haare mit Wasser ausgespült, sodann mit einem Handtuch frottiert, zur Frisur gelegt und getrocknet.
Als Ergebnis wird eine gleichmäßige, elastische und dauerhafte Verformung der Haare erhalten.

| Beispiel 4: Dauerverformungsmittel für gefärbtes Haar | |
|---|---|
| 12,0 g | Cysteinhydrochlorid |
| 4,2 g | Ammoniumthioglykolat |
| 6,0 g | Ammoniak (25-%ige wäßrige Lösung) |
| 3,0 g | 1,2-Propandiol |
| 3,0 g | 1,2-Butandiol |
| 3,0 g | Copolymer aus Vinylpyrrolidon und Dimethyl- aminoethylmethacrylat, mit Diethylsulfat quaternisiert (20-%ige wäßrige Lösung; Handelsname: GAFQUAT® 755 der GAF Corp., New York/USA) |
| 2,0 g | Ammoniumhydrogencarbonat |
| 0,8 g | Cocoamidopropylbetain |
| 2,0 g | diquaternäres Polysiloxan der Formel (I) (Handelsname:: ABIL- ®QUAT 3270 der Firma Th. Goldschmidt AG, Essen/BRD) |
| 0,1 g | Vinylpyrrolidon/Styrol-Mischpolymerisat (Handelsname:: ANTARA® 430 der GAF Corp., New York/USA) |
| 63,9 g | Wasser |
| 100,0 g | |

Der pH-Wert dieses Dauerverformungsmittels beträgt 8,2. Das Haar wird in der in Beispiel 4 beschriebenen Weise behandelt, wobei die Einwirkungszeit des Dauerverformungsmittels 15 Minuten beträgt.

Das so behandelte Haar verfügt über eine gleichmäßige Krause, deren Locken eine gute Elastizität und Sprungkraft aufweisen.

Als diquaternäres Polysiloxan (Handelsname: ABIL® -QUAT 3270) wurde in den Beispielen 1 bis 5 eine Verbindung der Formel (I) verwendet, bei der gilt:

M =
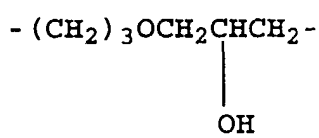

R4, R5 = $CH_3$
x = 3
R6 = NH-Rest
R7 = Kokosfettsäurerest
n = 10
$X^-$ = Acetatanion

Beispiel 5: Vergleichsversuche

Für die Vergleichsversuche wurden Zählhaarsträhnen, bestehend aus jeweils 100 Haaren gleicher Qualität, auf spiralförmige Wickler aufgewickelt und mit dem erfindungsgemäßen Mittel in üblicher Weise behandelt. Nach einer Einwirkungszeit des Dauerverformungsmittels von 10 Minuten bei 45 Grad Celsius wurden die Haare mit Wasser gespült und sodann oxidativ nachbehandelt. Nach dem Trocknen wurden die Haarsträhnen von den Wicklern genommen und in ein 40 Grad Celsius warmes Wasserbad gehängt.

Anschließend wurde die Gesamtlänge der Haarsträhne sowie die Länge der einzelnen Lockenwindungen in bestimmten Zeitabständen (nach 0, 10, 60, 120, 240, 360 Minuten) gemessen. Aus der Gesamtlänge der Haarsträhne wurde die Wellstabilität ("Curl Retention") nach folgender Formel berechnet:

$$\text{Wellstabilität [\%]} = \frac{(lo\text{-}l_t) \cdot 100}{(lo\text{-}l_1)}$$

mit

$l_o$ = Länge der gestreckten Haarsträhne
$l_t$ = Länge der ausgehängten Haarsträhne nach t = 0, 10, 60, 120, 240 oder 360 Minuten
$l_1$ = Länge der verformten, aufgewickelten Haar strähne

Die Wellstabilität ist ein Maß für die Wirksamkeit eines Dauerverformungsmittels und die Haltbarkeit einer Dauerwelle. Die Länge einer Lockenwindung wird im folgenden als Krausenstärke [mm/Locke] bezeichnet. Sie stellt ein gutes Maß für die Charakterisierung des Lockenbildes dar, da sie den optischen Eindruck eines Lockenbildes sehr gut wiedergibt. Ein kleiner Wert für die Krausenstärke bedeutet hierbei ein kleinlockiges Wellbild.

Die Wellstabilität und die Krausenstärke der mit einem mildalkalischen Dauerverformungsmittel der folgenden Zusammensetzung

| | |
|---|---|
| 11,9 g | Ammoniumthioglykolat |
| 5,0 g | Ammoniumhydrogencarbonat |
| 5,0 g | Polymer (a) bis (g) |
| 1,0 g | mit 4 Mol Ethylenoxid oxethylierter Laurylalkohol |
| 0,5 g | Ammoniak (25-%ige wäßrige Lösung) |
| 0,5 g | Cocoamidopropylbetain |
| 0,5 g | Parfümöl |
| 75,6 g | Wasser |
| 100,0 g | |

behandelten Haare wurde jeweils für den Haaransatz und die Haarspitzen gemessen.

Der pH-Wert des verwendeten Dauerverformungsmittels beträgt 8,3.

Als Polymere wurden hierbei die folgenden Verbindungen eingesetzt:

(a) Polyquaternium-6 (Poly(diallyldimethylammoniumchlorid)

(b) Polyquaternium-16 (Copolymer aus Vinylimidazoliummethochlorid und Vinylpyrrolidon)

(c) CROQUAT® WKP der Firma Croda/BRD (quaternisiertes Eiweißhydrolysat)

(d) Dow Corning® 929-Cationic Emulsion der Dow Corning Europe/Belgien (kationische Emulsion aus Amodimethicone, Tallowtrimonium Chloride und Nonoxynol-10)

(e) Pecosil SMQ-40 der Phoenix Chemical Inc./USA (Dimethicone Copolyol Phospho Myristyl Ammonium Chloride)

(f) Pecosil SWP Q 40 der Phoenix Chemical Inc. USA (quaternäres Eiweißhydrolysat/Dimethicone Copolyol Phosphat)

(g) Abil® -Quat 3270 (diquaternäres Polysiloxan gemäß Formel (I)).

In der nachfolgenden Tabelle sind die Ergebnisse der Messung der Wellstabilitäten nach einer Aushängezeit von t = 360 Minuten für die mit dem vorstehend beschriebenen Dauerverformungsmittel behandelten Haarsträhnen zusammengefaßt.

Tabelle 1

| Wellstabilitäten in Abhängigkeit des verwendeten Polymers | | | |
|---|---|---|---|
| Polymer | Wellstabilität im Bereich des Haaransatzes [%] | Wellstabilität im Bereich der Haarspitzen [%] | Differenz der Wellstabilität im Bereich der Haarspitzen und des Haaransatzes [%] |
| ohne Polymerzusatz | 39,8 | 43,4 | 3,6 |
| (a) | 36,9 | 51,8 | 14,9 |
| (b) | 38,1 | 49,2 | 11,1 |
| (c) | 41,1 | 46,9 | 5,8 |
| (d) | 37,2 | 46,9 | 9,7 |
| (e) | 38,7 | 43,8 | 5,1 |
| (f) | 44,1 | 47,1 | 3,0 |
| (g) | 47,0 | 45,0 | -2,0 |

Aus Tabelle 1 ist klar ersichtlich, daß bei Verwendung des erfindungsgemäßen Dauerverformungsmittel (g) der Unterschied zwischen den Wellstabilitäten im Bereich des Haaransatzes und der Haarspitzen am geringsten ist und keine "Überkrausung" der Haarspitzen stattfindet, sondern die Verformung der Haare im Bereich des Haaransatzes sogar geringfügig stärker ist als im Bereich der Haarspitzen.

Dieses Ergebnis wird durch die nachfolgende Abbildung 1 nochmals verdeutlicht.

**Abbildung 1: Differenz der Wellstabilitäten im Bereich der Haarspitzen und des Haaransatzes**

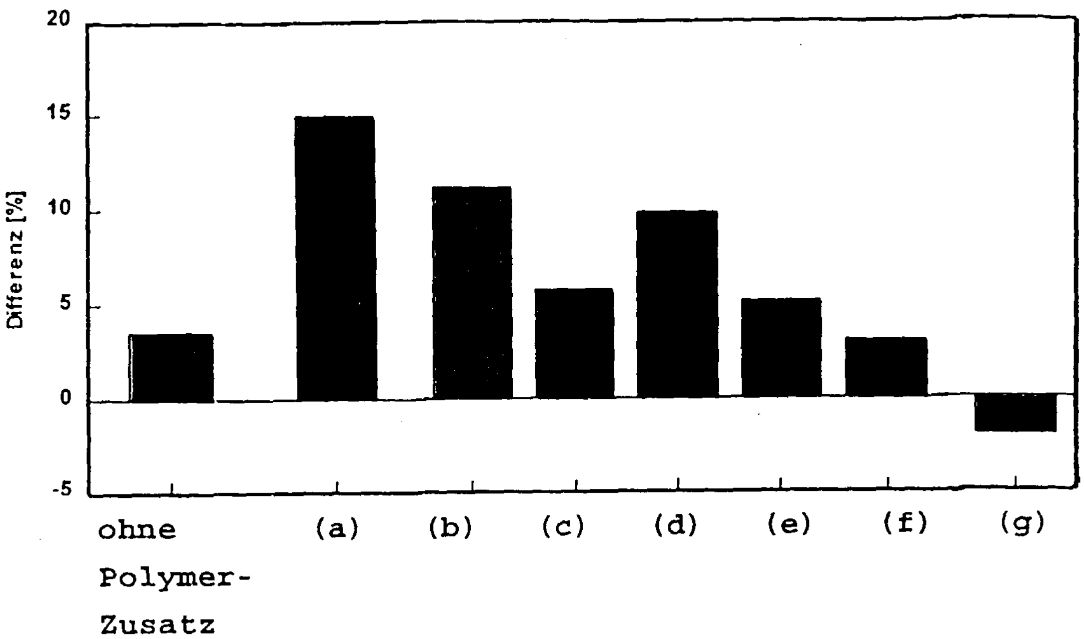

Im Gegensatz zu dem erfindungsgemäßen Mittel (g) zeigen die übrigen Dauerverformungsmittel je nach verwendetem Polymer eine mehr und weniger große Differenz der Wellstabilitäten im Bereich der Haarspitzen und des Haaransatzes, wobei diese Problematik insbesondere bei Verwendung von Polyquaternium-6 (a) zu beobachten ist. Infolge dieser Unterschiede der Wellstabilitäten im Bereich der Haarspitzen und des Haaransatzes wird bei den nicht-erfindungsgemäßen Mitteln ein unruhiges und ungleichmäßiges Verformungsergebnis erhalten, welches teilweise sogar deutlich schlechter ist als bei Verwendung eines Polymer-freien Dauerverformungsmittels.

Eine weitere, exakte Möglichkeit der Bewertung des Verformungsergebnisses im Bereich des Haaransatzes und der Haarspitzen ist neben der Wellstabilität die Krausenstärke. Die nachfolgende Tabelle 2 gibt einen Überblick über die ermittelten Differenzen bezüglich der Krausenstärke für den Haaransatz und für die Haarspitzen.

Tabelle 2:

| Differenz der Krausenstärken im Bereich des Haaransatzes und der Haarspitzen | |
|---|---|
| Polymer | Differenz der Krausenstärke [mm] |
| ohne Polymerzusatz | 3,58 |
| (a) | 8,83 |
| (b) | 5,95 |
| (c) | 4,69 |
| (d) | 5,40 |
| (e) | 6,43 |
| (f) | 4,12 |
| (g) | 1,60 |

Der Sachverhalt soll durch die nachfolgende Abbildung 2 verdeutlicht werden.

Abbildung 2: Differenz der Krausenstärke im Bereich des Haaransatzes und der Haarspitzen

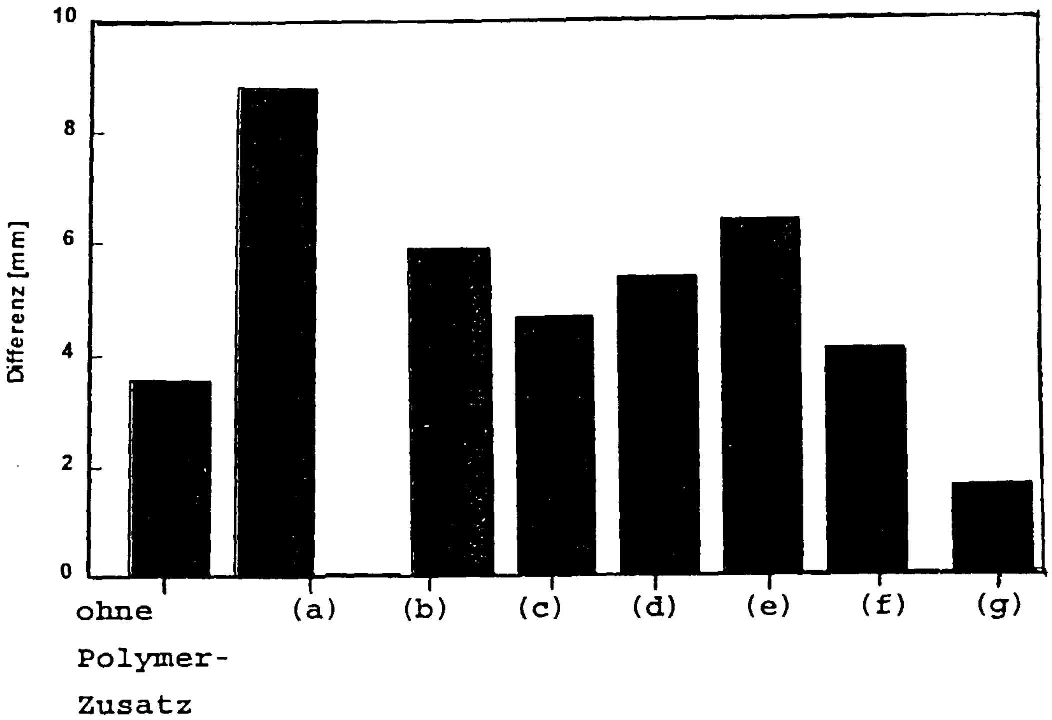


Aus Tabelle 2 und Abbildung 2 ist klar ersichtlich, daß bei den nicht-erfindungsgemäßen Verformungsmitteln die Krausenstärke im Bereich der Haarspitzen deutlich kleiner ist als im Bereich des Haaransatzes. Dies bedeutet, daß das Lockenbild vom Haaransatz zu den Haarspitzen immer kleinlockiger wird. Ein derartiges Lockenbild wirkt sehr unruhig und erzeugt insbesondere durch die Kleinlockigkeit der Haarspitzen den Eindruck einer Überkrausung der Haare.

Im Gegensatz hierzu ist die Krausenstärke bei Verwendung des erfindungsgemäßen Verformungsmittels (g) im Bereich des Haaransatzes und der Haarspitzen nahezu identisch, wodurch ein gleichmäßiges Lockenbild erhalten wird, das auch ein gutes Wellergebnis im Bereich des Haaransatzes aufweist.

Alle in der vorliegenden Anmeldung aufgeführten Prozentangaben stellen, soweit nicht anders angegeben, Gewichtsprozent dar.

## Patentansprüche

1. Mittel zur dauerhaften Verformung von Haaren auf der Basis einer keratinreduzierenden Verbindung, dadurch gekennzeichnet, daß es ein diquaternäres Polysiloxan der allgemeinen Formel (I)

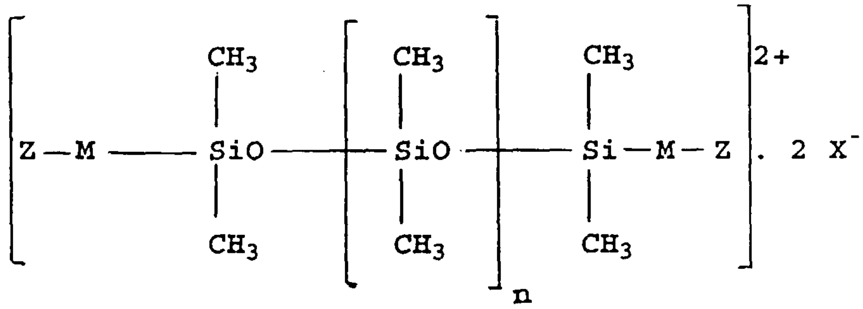 (I),

wobei
Z der Rest

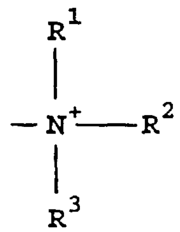 ; 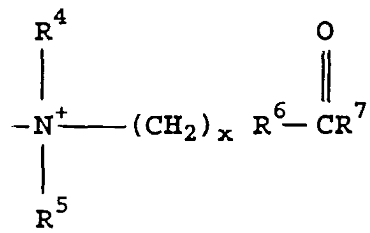 oder 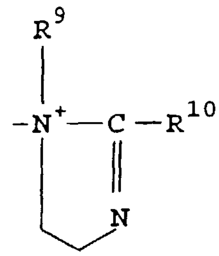

ist,

$R^1$, $R^2$, $R^3$ = Alkylreste mit 1 bis 22 Kohlenstoffatomen oder Alkenylreste mit 2 bis 22 Kohlenstoffatomen, wobei die Alkyl- oder Alkenylreste Hydroxylgruppen aufweisen können und mindestens einer der Reste $R^1$, $R^2$, $R^3$ mindestens 10 Kohlenstoffatome aufweist,

$R^4$, $R^5$, $R^7$, $R^9$, $R^{10}$ = Alkylreste mit 1 bis 22 Kohlenstoffatomen oder Alkenylreste mit 2 bis 22 Kohlenstoffatomen, wobei die Alkyl- oder Alkenylreste Hydroxylgruppen aufweisen können,

$R^6$ = -O- oder $NR^8$-Rest, $R^8$ = Alkyl- oder Hydroxyalkylrest mit 1 bis 4 Kohlenstoffatomen oder Wasserstoffrest,

x = 2 bis 4,

M = ein zweiwertiger Rest, ausgewählt aus der Gruppe

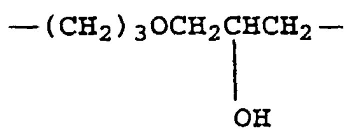 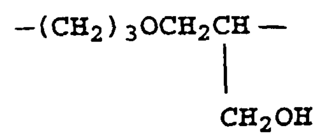

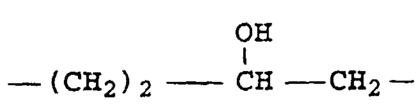 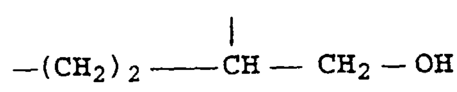

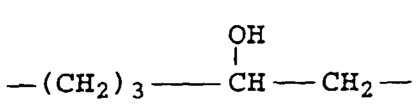 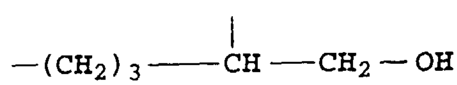

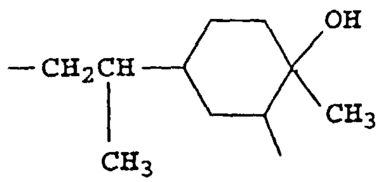

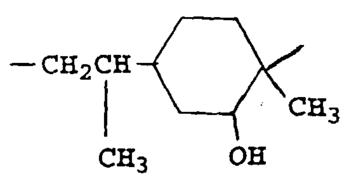

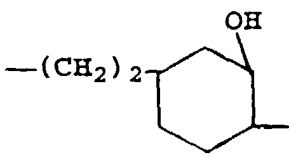

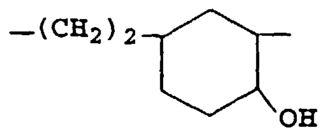

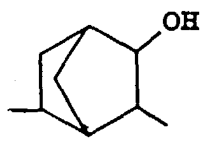

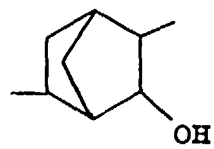

wobei das N-Atom des Restes Z mit dem Rest M über das zur C-OH-Gruppe im Rest M benachbarte Kohlenstoffatom verbunden ist,

n = eine Zahl von 0 bis 200,
$X^-$ anorganisches oder organisches Anion, das von einer üblichen physiologisch verträglichen Säure HX herrührt,

enthält.

**2.** Mittel gemäß Anspruch 1, dadurch gekennzeichnet, daß die keratinreduzierende Verbindung ausgewählt ist aus Thioglykolsäure oder deren Salzen, Thiomilchsäure oder deren Salzen, 2-Hydroxy-3-mercaptopropionsäure oder deren Salzen, Cystein oder dessen Salzen und Derivaten; Cysteamin oder dessen Salzen und Derivaten, und Schweflige Säure oder deren Salzen.

**3.** Mittel nach einem der Ansprüche 1 oder 2, dadurch gekennzeichnet, daß das diquaternäre Polysiloxan ausgewählt ist aus Verbindungen der Formel (I) mit M = $-(CH_2)_3-O-CH_2-CH\,(OH)-CH_2-$;

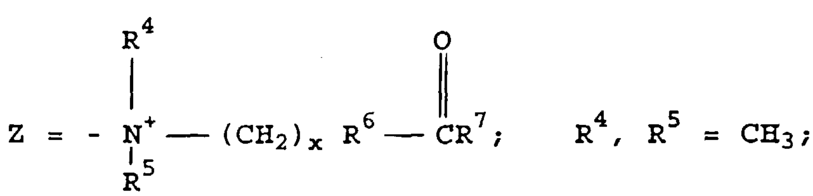

$R^6$ = - NH-Rest; $R^7$ = Kokosfettsäurerest; x = 3;
n = 10 oder 30 und $X^-$ = Acetatanion.

**4.** Mittel nach einem der Ansprüche 1 bis 3, dadurch gekennzeichnet, daß das diquaternäre Polysiloxan der Formel (I) in einer Menge von 0,02 bis 5 Gewichtsprozent enthalten ist.

**5.** Verfahren zur dauerhaften Verformung von Haaren, bei dem man das Haar bevor und/oder nachdem man es in

die gewünschte Form bringt mit einem Verformungsmittel behandelt, mit Wasser spült, gegebenenfalls zur Wasserwelle legt und sodann trocknet, dadurch gekennzeichnet, daß man als Verformungsmittel ein Mittel gemäß Anspruch 1 oder 2 verwendet.

## Claims

1. Agent for the permanent shaping of hair based on a keratin-reducing compound, characterised in that it contains a diquaternary polysiloxane of the general formula (I)

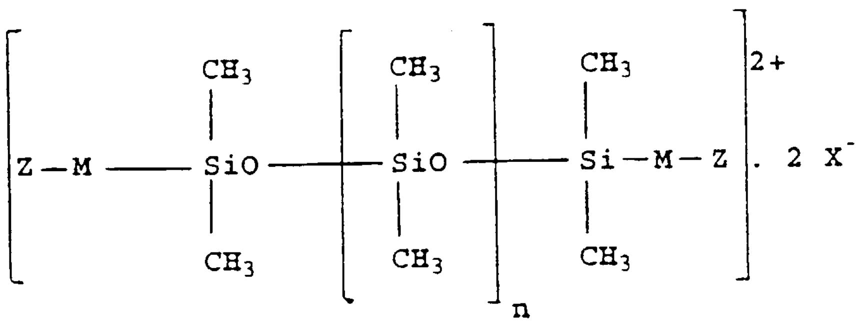

(I),

wherein
Z is the residue

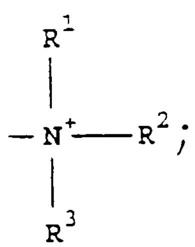

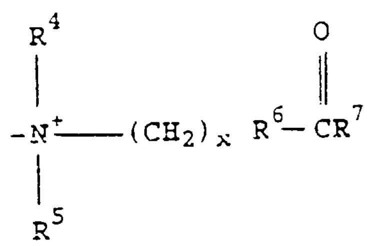

or

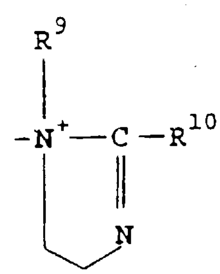

$R^1$, $R^2$, $R^3$ = alkyl residues having from 1 to 22 carbon atoms or alkenyl residues having from 2 to 22 carbon atoms, wherein the alkyl or alkenyl residues may contain hydroxy groups and at least one of the residues $R^1$, $R^2$, $R^3$ contains at least 10 carbon atoms,
$R^4$, $R^5$, $R^7$, $R^9$, $R^{10}$ = alkyl residues having from 1 to 22 carbon atoms or alkenyl residues having from 2 to 22 carbon atoms, wherein the alkyl or alkenyl residues may contain hydroxy groups,
$R^6$ = an -O- or $NR^8$ residue, $R^8$ = an alkyl or hydroxyalkyl residue having from 1 to 4 carbon atoms or a hydrogen residue,
x = 2 to 4,
M = a divalent residue selected from the group

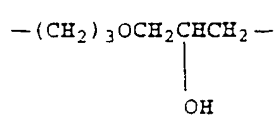

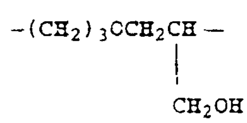

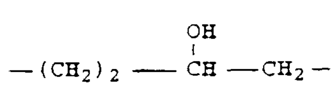

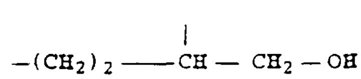

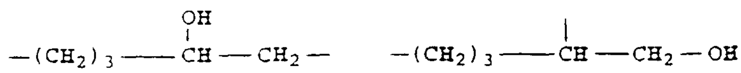

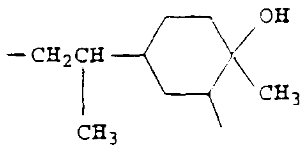

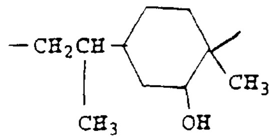

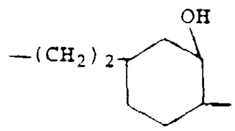

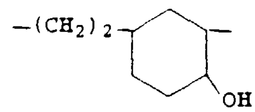

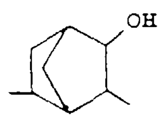

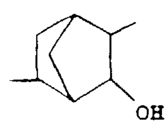

wherein the nitrogen atom of the resudue Z is bonded to the residue M by way of the carbon atom adjacent to the C-OH group in the residue M,

n = an integer from 0 to 200,
$X^-$ = an inorganic or organic anion which originates from a customary physiologically compatible acid HX.

2. Agent according to Claim 1, characterised in that the keratin-reducing compound is selected from thioglycolic acid or its salts, thiolactic acid or its salts, 2-hydroxy-3-mercaptopropionic acid or its salts, cysteine or its salts and derivatives, cysteamine or its salts and derivatives, and sulphurous acid or its salts.

3. Agent according to any one of Claims 1 or 2, characterised in that the diquaternary polysiloxane is selected from compounds of formula (I) wherein
M =$-(CH_2)_3-O-CH_2-CH(OH)-CH_2-$;

Z = 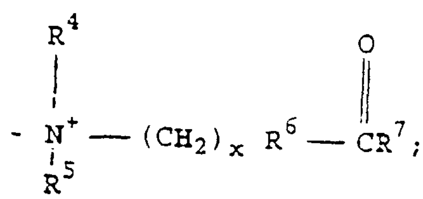

$R^4$, $R^5$ = $CH_3$;
$R^6$ = a -NH residue; $R^7$ = a coconut fatty acid residue; x = 3;
n = 10 or 30 and $X^-$ = an acetate anion.

4. Agent according to any one of Claims 1 to 3, characterised in that the diquaternary polysiloxane of the formula (I) is present in an amount of from 0.02 to 5% by weight.

**5.** Process for the permanent shaping of hair, in which the hair, before and/or after it has been brought into the desired shape, is treated with a shaping agent, rinsed with water, optionally arranged in a water wave and then dried, characterised in that an agent according to Claim 1 or 2 is used as the shaping agent.

## Revendications

**1.** Agent de déformation permanente des cheveux, à base d'une combinaison réduisant la kératine, caractérisé en ce que l'on utilise un polysiloxane diquaternaire de formule générale (1)

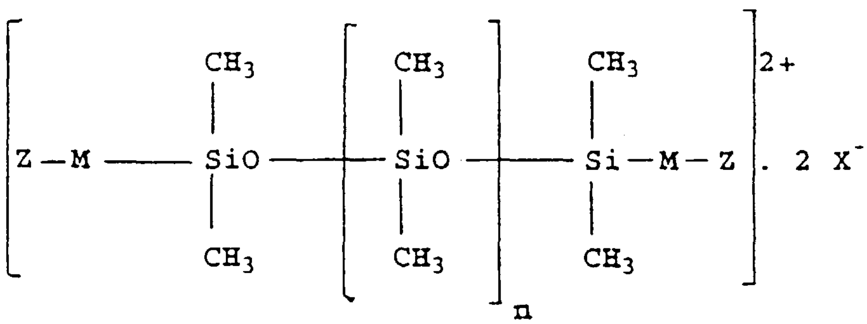

(I),

dans laquelle Z est le reste

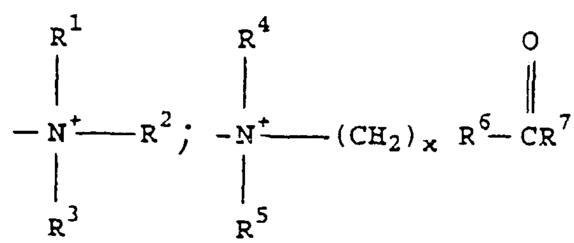

ou

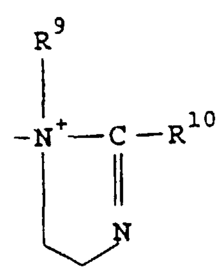

$R^1$, $R^2$, $R^3$ = reste alkyle contenant de 1 à 22 atomes de carbone ou restes à alkényle contenant de 2 à 22 atomes de carbone, les restes alkyle ou alkényle pouvant présenter des groupes hydroxyle et au moins l'un des restes $R^1$, $R^2$, $R^3$ présentant au moins 10 atomes de carbone.

$R^4$, $R^5$, $R^7$, $R^9$, $R^{10}$ = restes alkyle contenant de 1 à 22 atomes de carbone ou restes alkényle contenant de 2 à 22 atomes de carbone, les restes alkyle ou alkényle pouvant présenter des groupes hydroxyle.

$R^6$ = -0- ou un reste $NR^8$, $R^8$ = reste alkyle ou hydroxyalkyle et contenant de 1 à 4 atomes de carbone ou un reste hydrogène,

x = 2 à 4.

M = un reste bivalent, choisi dans le groupe

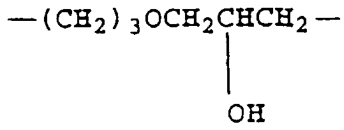

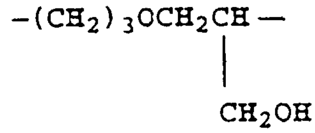

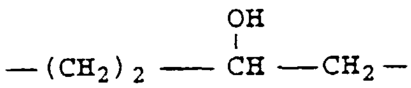

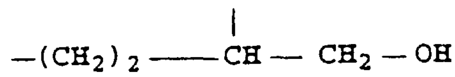

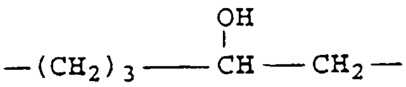

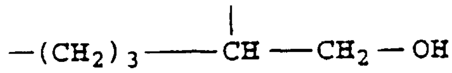

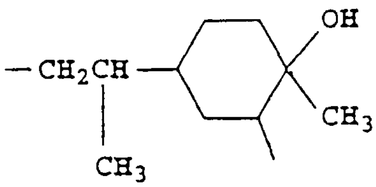

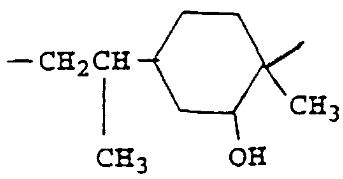

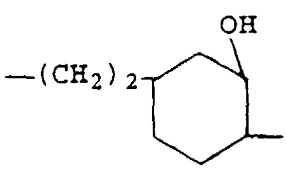

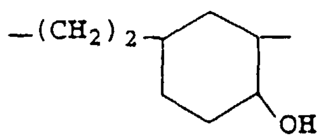

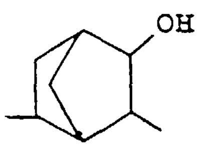

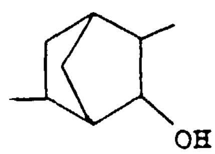

l'atome N du reste Z étant combiné au reste par l'intermédiaire de l'atome de carbone voisin du groupe C-OH dans le reste,

n = un nombre compris dans la plage allant de 0 à 200

$X^-$ = un ion anorganique ou organique, provenant d'un acide HX compatible physiologiquement usuel.

**2.** Agent selon la revendication 1, caractérisé en ce que la combinaison réduisant la kératine est choisie dans le groupe composé de l'acide thioglycolique ou ses sels, l'acide thiolactique ou ses sels, l'acide 2-hydroxy-3-mercaptopropionique ou ses sels, la cystéine ou ses sels et les dérivés, la cystéamine ou ses sels et les dérivés et le l'acide sulfureux ou ses sels.

**3.** Agent selon l'une des revendications 1 ou 2, caractérisé en ce que le polysiloxane diquaternaire est choisi dans les combinaisons de la formule (I) avec

M = $-(CH_2)_3-O-CH_2-CH(OH)-CH_2-$;

Z =

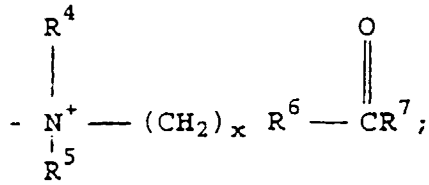

$R^4$, $R^5$ = $CH_3$

$R^6$ = -NH-reste; $R^7$ = reste de l'acide de l'huile de coprah; x = 3;

n = 10 ou 30 et $X^-$ = anion d'acétate.

**4.** Agent selon l'une des revendications 1 à 3, caractérisé en ce que le polysiloxane diquaternaire de formule (I) et contenu en une quantité comprise allant de la plage allant de 0,02 à 5 pour cent en poids.

**5.** Procédé de déformation permanente des cheveux, pour lequel on traite les cheveux, avant et/ou après les avoir mis à la forme souhaitée, avec un agent de déformation, on rince les cheveux, le cas échéant à grandes eaux et l'on sèche ensuite, caractérisé en ce que l'on utilise comme agent de déformation un agent selon la revendication 1 ou la revendication 2.